# EUROPEAN PATENT SPECIFICATION

(11) **EP 1 436 420 B1**
(45) Date of publication and mention of the grant of the patent: **25.04.2007**
(21) Application number: 02765125.6
(22) Date of filing: 03.10.2002
(51) Int. Cl.: C12Q 1/68

(54) **TEST STRIP ASSAY SYSTEM AND METHOD FOR THE DETECTION OF SPECIFIC NUCLEIC ACID SEQUENCES**
TEST-STREIFE-PROBESYSTEM UND VERFAHREN ZUR NACHWEIS VON SPEZIFISCHEN NUKLEINSÄURESEQUENZEN
SYSTEME DE BANDELETTE REACTIVE A TREMPER ET METHODE D'ESSAI POUR DETECTER ET/OU ANALYSER DES SEQUENCES D'ACIDE NUCLEIQUE SPECIFIQUES

(30) Priority: 16.10.2001 GR 2001100478
(43) Date of publication of application: 14.07.2004
(73) Proprietor: Soufla, Giannoula, 15771 Zografou Attikis, Athens (GR)
(72) Inventor: Soufla, Giannoula, 15771 Zografou Attikis, Athens (GR)
(86) International application number: PCT/GR2002/000052
(87) International publication number: WO 2003/033735

(56) References cited:
- WO-A-01/00876
- WO-A-90/01564
- WO-A-92/07096
- FONG WHALLEY K ET AL: "Rapid solid-phase immunoassay for detection of methicillin-resistant Staphylococcus aureus using cycling probe technology." JOURNAL OF CLINICAL MICROBIOLOGY, vol. 38, no. 7, July 2000 (2000-07), pages 2525-2529, XP002202273 ISSN: 0095-1137
- GRAVITT P E ET AL: "Genotyping of 27 human papillomavirus types by using L1 consensus PCR products by a single-hybridization, reverse line blot detection method." JOURNAL OF CLINICAL MICROBIOLOGY, vol. 36, no. 10, October 1998 (1998-10), pages 3020-3027, XP002202274 ISSN: 0095-1137

## Description

The present invention concerns an analytical device, specifically a dipping test strip assay system with dried reagents, as well as an assay method for the detection or/and determination of specific nucleic acid sequences of clinical significance with the use of said test strip.

More specifically, the present invention concerns an one step assay system which is particularly useful for sensitive rapid test diagnostic devices: a dipping test strip assay system with dried reagents, as well as the development of hybridization assays on said dipping test strip, with which it is possible to detect the presence or absence of a specific nucleic acid sequence in a biological sample, with great ease and speed.

Dipping test strips are systems consisting of different kinds of materials and reagents, dried or immobilized on these materials, supported by a flexible polystyrene film, which is actually used as the base for the construction of the system. The system consists of at least four distinct/discernible areas: a) the area or part of the test strip which comes into contact with the sample or with the assay-development solution on the strip, b) the area or part of the test strip on which the reagent bearing or responsible for the visible signal is dried, c) the area or part of the test strip on which the signal is developed (membrane area), d) the area or part of the test strip with the task to withhold the excess of the liquid sample or liquid containing the analyte. In certain cases the areas a) and b) or b) and c) may not be discernible. The dipping test strip assay system of the present invention consists of the four distinct areas mentioned above and bears at area b): color microspheres, and at area c): immobilized reagents in two discernible zones (lanes or dots) indicating the presence or absence of the detected nucleic acid and confirming the completion [as well as the correct use and good condition of the dipping test strip] of the assay respectively.

The presence or absence, and the determination of the quantity of a specific nucleic acid sequence gives answers of major importance to issues investigated by microbiology, virology, genetics and biomedical research in general. The great evolution of Molecular Biology techniques and the many specific applications of the Polymerase Chain Reaction (PCR) urged the development of many techniques for the detection and the determination of the specific products of the PCR reaction.

Techniques comprising agarose or polyacrylamide gel electrophoresis for example: SSCP, DGGE, RFLP are very sensitive but have certain disadvantages such as the demand for trained personnel, long total analysis time, and the use of harmful or irritating reagents.

The techniques that take advantage of DNA/DNA, RNA/RNA or RNA/DNA hybridization for the detection of specific nucleic acid sequences are widely used. The replacement of the originally used radiolabeled probes by non isotopic probes for the detection of the hybrids has led to the surpassing of former disadvantages such as the use of harmful radiolabeled reagents with limited lifetime, without a compromise regarding the sensitivity or the specificity of the assay. Techniques of the above kind comprising immobilization of oligonucleotides on solid supports, such as nylon or nitrocellulose membranes (known as dot-blot and reverse dot-blot) are sensitive and robust, but they are unsatisfactory as regards the speed and ease of the analysis. Hybridization techniques comprising direct or indirect immobilization of oligonucleotides to polystyrene ELISA microtiter plates, have all the previously mentioned advantages of sensitivity, specificity and robustness, with the addition of susceptibility to automation and capability of simultaneous analysis of multiple samples, but they have the disadvantage of being heterogeneous which actually means that the analysis requires multiple steps.

Total analysis time is compared to that of the dot-blot and reverse dot-blot technique.

The use of one step rapid test systems (dipstick or lateral flow tests) during the 80's was revolutionary in the field of clinical immunoassays offering appealing advantages such as robustness, specificity, simplicity and ease of analysis, speed and low cost. Applications of the above systems regard testing for pregnancy, sexually transmitted diseases, food testing, toxins, bacteriological infections; environmental control, biological agents, allergen detection etc. However, in some cases the signals from current dipstick or lateral flow rapid tests are not sensitive enough to provide a qualitative or quantitative result, especially when the concentration of the substance to be detected is already extremely low in the sample, as for example in detection of HIV antibodies in saliva or blood, or other viral or bacterial infections in urine or blood which indicate the onset of a particular disease. In the latter case, the detection of viruses or bacteria at the DNA or RNA level has many advantages over the determination of the immunological response of the patient's immune system, such as diagnosis at an early stage of a virological/bacterial infection (even before the response of the immune system), detection of viral/bacterial DNA at low concentrations (low detection limit) with the sensitivity and specificity that characterize the Molecular Biology techniques, ability to monitor the effectiveness of a patients' therapy or treatment. The Molecular Biology techniques however, which are also currently used in the clinical laboratories worldwide for mutation detection, for the molecular diagnosis of genetic diseases, as well as the molecular diagnosis of cancer (breast cancer, prostate cancer etc), are inferior to dipstick and lateral flow tests as far as the speed and ease of use is concerned.

The present invention provides a one step assay system (a dipping test strip), which employs Molecular Biology methods and techniques, specifically the hybridization of complementary nucleic acid sequences, and combines the advantages of the molecular analysis with the simplicity, speed of analysis, and the low cost of rapid tests.

According to the present invention, this is achieved by means of a dipping test strip assay system with dried reagents consisting of the four distinct areas: a), b), c) and d) mentioned above, characterized in that area b) bears dried colloidal gold particles conjugated to an oligonucleotide, and in that area c) bears the protein Streptavidin and an oligonucleotide of complementary sequence to the oligo conjugated on the gold particles of area b), that are immobilized in two discernible zones (lanes or dots). The present invention also provides a method for the construction of said dipping test strip system, as well as an assay method for the detection and/or determination of specific nucleic acid sequences by means of developing particular hybridization assays on said test strip system, characterised in that an oligonucleotide-probe is used one part of which is complementary to the sequence of nucleic acid to be detected and another part is of complementary sequence to the oligo conjugated on the gold particles, as well as in that a visible signal is produced, based on hybridization of complementary nucleic acid sequences, indicative of the presence or absence of said specific nucleic acid sequence at a detection site on a support (area c) where the protein streptavidin is immobilised, and a visible signal (based on hybridization) indicative upon the completion of the assay (the sample along with the colloidal gold particles have reached the detection point) is produced at a control site on a support (area c) characterized in that an oligonucleotide is immobilized on said site.

The dipping test strip assay system with dried reagents of the present invention has many advantages. The fact that the protein streptavidin is immobilized at the detection site at area c), actually means that either a Biotinylated oligonucleotide is used as a probe for the specific nucleic acid sequence to be detected, or the specific nucleic acid to be detected is Biotinylated; it depends on the particular hybridization protocol that is developed on the strip. In this way we take advantage of the biotin-streptavidin bridge, which is very stable (K= 10¹⁵ L/mol), with the result of increased sensitivity in the assay where it is being employed, comparable to the sensitivity of radiolabeled probes. Using this technology, the assay is also characterized by specificity since the detection is based on hybridization of specific nucleic acid sequences. Moreover, the analysis time is drastically reduced from two or three hours required upon the quickest molecular biology techniques currently used to ten or twenty minutes, the analysis is very simple, and the former use of many hazardous materials and reagents is avoided.

The production cost of such a dipping test strip assay system is remarkably low.

Finally, equally important advantage of the dipping test strip assay system is its capability for universal use for the detection of many different nucleic acid sequences, with also the flexibility of the development of alternative hybridization protocols on it, by a single modification regarding the use of the appropriate specific probe for each nucleic acid to be detected, which is described below.

The invention will now be particularly described by way of example with reference to the accompanying diagrammatic drawings in which:
Fig.1 is a schematic drawing of an analytical test device illustrating the arrangement of the four distinct /discernible areas that consist the dipping test strip system in accordance with the present invention.
Fig.2 shows a schematic drawing illustrating the hybridization assay configurations developed on said test strip system that are responsible for providing a visible signal indicative of the presence or absence of a specific nucleic acid sequence in a sample, according to the present invention

For the convenience of the reader the same reference numbers are used in the following examples as in the drawings.

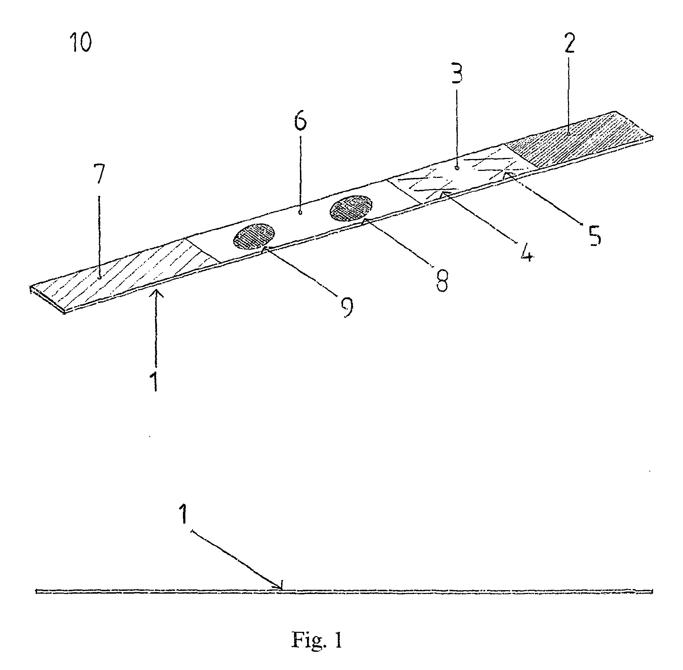

FIG. 1 shows a schematic drawing illustrating the dipping test strip system with dried reagents (10), which is supported by a flexible polystyrene film (1) that is actually used as the base for the construction of the system. The system (10) consists of at least four distinct areas of different materials and with a specific function: a) the area or part of the test strip which comes into contact with the sample or with the assay-development solution on the strip (2), b) the area or part of the test strip on which the detection reagent bearing or responsible for the optical signal is dried (3), c) the area or part of the test strip on which the signal is developed (membrane area) (6), d) the area or part of the test strip with the task to withhold the excess of the liquid sample or liquid containing the analyte (7). The area or part a) of the test strip which comes into contact with the sample or with the assay-development solution on the strip (2), constitutes of a porous element, usually cotton fiber, and has the property of absorbing quickly a large quantity of the assay-developing solution permitting however it's quantitative transfer to area b) (3) of the strip. The area (3) is constituted of glass fiber elaborated in a way that is capable of withholding the detection reagent dried upon, but also permitting it's release towards the membrane area when it is being rehydrated by the moving assay-development solution. The membrane area (6) comprises a nitrocellulose membrane or a suitably modified membrane to possess nitrocellulose functionality (nitro- groups on it's surface). Typical membranes that may be used in the system of the present invention are nitrocellulose membranes with pore sizes ranging from 5µm to 8 µm or 12 µm, depending on the particular application, (e.g. Schleicher& Schuell AE 98, AE 99, AE 100), as well as the polyethersulfon modified membrane to have nitrocellulose functionality: Predator (Pall & Gelman) with pore size 0,45µm. The area (7) (upper part of the system) is consisted of a strongly absorbent material comprising mainly cotton fiber.

According to the present invention, initially the protein Streptavidin (8) is immobilized on the membrane at a quantity of at least 0,5 µg (8333 fmol) diluted in a phosphate buffer 15mM pH 7,2 (streptavidin application solution), as well as an oligonucleotide (9) having length of at least 60 (sixty) bases and preferably between 60 and 100 bases the most, is also immobilized on the membrane in a way that forms either a dot or a lane and at a quantity of at least 500 fmol. The length of the oligonucleotide being immobilized is of substantial importance to the success of the immobilization. The immobilization of the protein Streptavidin on the membrane takes place at a distance of 1 cm (8) from the edge of the membrane that comes into contact with the part (3) of the strip, while the oligonucleotide is immobilized at a distance of 1,5 cm from the same edge of the membrane. The immobilization is carried out by the application of the reagent at the selected area of the membrane followed by baking in an oven of controlled temperature and humidity, at 70-80°C for a period of time ranging from 30 to 120 minutes depending on the type of the membrane being used. The assembly of the system of the strip on the flexible polystyrene film follows said immobilization.

According to the present invention, colloidal gold particles are applied and dried on area (3) of the strip assay system and more concretely at the lower half (5) of area (3) [towards the part of the strip that comes into contact with the assay-development solution], wherein said colloidal gold particles are conjugated to an oligonucleotide of certain length and concretely having the length of 60 (sixty) to 100 (a hundred) bases, and of complementary sequence to the oligo previously immobilized (9) on the membrane. The oligonucleotide to be conjugated to the colloidal gold particles is modified to possess an SH group at it's 5' end, and the conjugation is carried out by the method described by Mirkin C.A., Letsinger R.L., Mucic R.C., and Storhoff J.J., in Nature **382**, 607-609 (1996). According to said method of conjugation the oligonucleotide is added to a quantity of colloidal gold particles and incubated at 4°C for 56 hours. The ratio (moles colloidal gold particles)/(moles oligonucleotide) maintained at the conjugation procedure is at least 1/500 and preferably between 1/500 and 1/1000. Said conjugated colloidal gold particles are dried on area (3) by baking at 40 °C for two hours or preferably by allowing the particles' application solution to air dry in an area of confined humidity. The application buffer of said conjugated particles about to be dried on area (3) of the test strip, contains Bovine Serum Albumin (BSA) 5% w/v, Sucrose 5% w/v, and Tween 20 at a quantity between 0,01% and 0,1% w/v and preferably 0,05%w/v. The gold particles used in the present invention for conjugation with oligonucleotides may have a diameter of 20 nm or 30 nm or 40 nm.

At the upper half (4) of area (3) of the dipping test strip of the present invention, the sample suspected to contain the analyte (DNA) is applied, said DNA is previously denatured by heating at 95°C for 5min and prehybridized for at least 15-20 min with one or two oligonoucleotides-probes at a temperature depending on the Tm of the probes and at suitable conditions of ionic strength, pH and the appropriate buffer (J.Mol.Biol. **98**, 503 (1975), Proc.Natl.Acad Sci. USA **76**, 3683 (1979)). Said prehybridization is highly recommended because it increases the sensitivity of the hybridization assay on the strip and therefore the sensitivity of the detection with the use of the present invention. For each hybridization assay for the detection of a specific nucleic acid with the dipping test strip of the present invention, at least 5µl of the PCR product is necessary to be used.

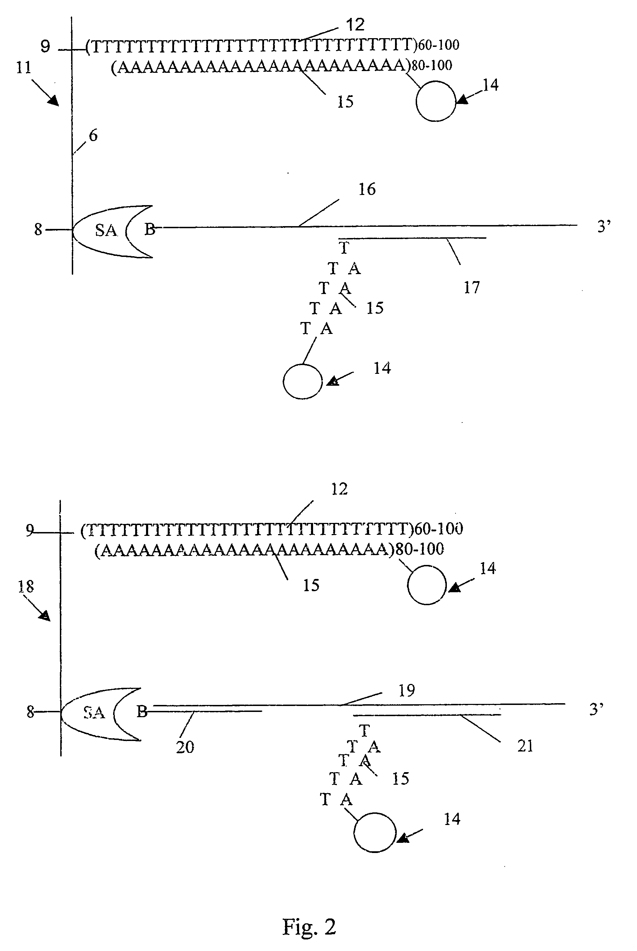

Fig.2 shows a schematic drawing illustrating the hybridization assay configurations related to appropriate hybridization protocols developed on said test strip system that are responsible for providing a visible signal indicative of the presence or absence of a specific nucleic acid sequence in a sample, according to the present invention.

According to the hybridisation protocol I (11) of the present invention, 500 fmol of an oligonucleotide (12) having the length of 60 to 100 Thymine bases (T), is immobilised on area (9) of the membrane, as well as 0,5 µg at least of the protein Streptavidin (SA) (13) in a phosphate buffer 15mM pH 7,2 is also immobilized on area (8) of the membrane. The oligonucleotide, which is immobilized on the membrane, may alternatively consist of a random sequence of at least 20 nucleotide bases, followed by a 40-80 bases sequence of Thymines (T) that is tailed at the 3' end of the oligo of random sequence with the use of the enzyme: terminal transferase. An oligo of 80-100 Adenines (A) (15) is conjugated on the surface of colloidal gold particles (14). The target DNA (16) is the product of a PCR reaction in which one of the primers is Biotinylated, so that one strand of the PCR product is Biotinylated at its 5' end. An oligo-probe (17) consisting of 20-24 bases of complementary sequence to a specific region of the Biotinylated PCR product is also needed, wherein said probe carries additionally 80-100 bases of Thymine (T) at it's 5' or 3' end. The probe (17) may carry such number (80-100) of Thymines (T) by having it synthesized in that way, or the 20-24mer may be tailed at it's 3' end with the use of the enzyme: terminal transferase. Heating at 95 °C for 5 min denatures the sample containing the PCR product to be detected, which is then prehybridized with the specific nucleic acid probe (17) for at least 15-20 min at a temperature depending on the Tm (melting point) of the probe and at suitable conditions of ionic strength, pH and buffer (J.Mol.Biol. **98**, 503 (1975), Proc.Natl.Acad Sci. USA **76**, 3683 (1979)). At least 1500-2000 fmol and preferably 1500-5000 fmol of probe (17), is used for hybridization with 5 µL (100-200 fmol/µL) of Biotinylated PCR product.

According to the hybridisation protocol II of the present invention, 500 fmol of an oligonucleotide (12) having the length of 60 to 100 Thymine bases (T), is immobilised on area (9) of the membrane, as well as 0,5 µg at least of the protein Streptavidin (SA) (13) in a phosphate buffer 15mM pH 7,2 is also immobilized on area (8) of the membrane. The oligonucleotide, which is immobilized on the membrane may alternatively consist of a random sequence of at least 20 nucleotide bases, followed by a 40-80 bases sequence of Thymines (T) that is tailed at the 3' end of the oligo of random sequence with the use of the enzyme: terminal transferase. An oligo of 80-100 Adenines (A) (15) is conjugated on the surface of colloidal gold particles (14). The target DNA (19) is the product of a PCR reaction in which the specific primers used are not labeled, so that the reaction yields a PCR product that is not labeled.

Two oligonucleotides-probes consisting of 20-24 bases of complementary sequence to two discernible areas of the same strand of the PCR product are also needed. One of said oligo-probes is labeled with Biotin (20) at its 3' or 5' end, while the other oligo-probe (21) carries additionally 80-100 bases of Thymine (T) at it's 5' or 3' end. The labeling of the probe (20) with Biotin may be either at its 5' or at its 3' end, said labeled probe may be synthesized in that particular way or labeling may take place at the 3' end of the 20-24mer by the use of the enzyme: terminal transferase and a Biotin-labeled nucleotide such as Biotin-14-dATP. The double stranded DNA-target (19) is denatured by heating at 95°C for 5 min and then prehybridized with the two specific nucleic acid probes (20,21) for at least 15-20 min at a temperature depending on the melting point (Tm) of the two probes and at suitable conditions of ionic strength, pH, and buffer (J.Mol.Biol. **98,** 503 (1975), Proc.Natl.Acad Sci. USA **76**, 3683 (1979)). For the hybridization of 5 µL PCR product (100-200 fmol/µL), at least 1500-2000 fmol of each of the two probes (20,21) is used and preferably 1500-5000 fmol the most of each of the probes.

The protocols I and II can also be developed on the strip assay system of the present invention by mutual exchange of the oligonucleotides being immobilized on the membrane and the surface of the colloidal gold particles respectively. Specifically an oligonucleotide of 60-100 Adenines (A) can be immobilized on the membrane, while a complementary sequence oligonucleotide of 80-100 Thymines (T) is conjugated to the surface of the colloidal gold particles. In this case the 20-24mers oligonucleotides-probes used in the hybridization assays of both protocols I and II must carry additionally 80-100 bases of Adenine (A) instead of Thymine (T) at their 5' or 3' end.

Following the application of the sample suspected to contain the specific nucleic acid sequence at the upper half (4) of area (3) of the dipping test strip of the present invention, the strip is immersed in a tube or vessel containing the hybridization solution that travels along the strip drawn by capillary forces and encounters the dried colloidal gold particles which it rehydrates and induces to the membrane area at first, and then to the upper part of the test strip that has the task to withhold the excess of the liquid sample. The constitution of the hybridization solution is such that it permits the hybridization of complementary nucleic acid sequences as well as the formation of the Biotin-Streptavidin bond. Specifically, the hybridization solution contains a buffer pH 7,5-7,6, Bovine Serum Albumin (BSA) at least 5% w/v and preferably 5%-7% w/v, NaCl 150 mM, glycerol at least 15% v/v and preferably 15%-30% v/v, and a non-ionic surfactant or detergent at a quantity at least 0,1% v/v and preferably 0,1%-5% v/v. A suitable buffer is PBS pH 7,5 or maleic acid buffer pH 7,6 and a suitable non-ionic surfactant is Tween 20,or Triton X-100 or any other non-ionic detergent of the same category. The glycerol in the hybridization solution has double function: it increases the viscosity of the solution which then moves along the test strip driven by capillary forces more slowly allowing the hybridization to take place more effectively, thus affecting the success of the assay, and secondly it has the ability of promoting the hybridization efficiency. Other substances that promote the hybridization efficiency and can be used instead of glycerol or in addition to glycerol in the hybridization solution are polymers such as PVP (polyvinylopyrrolidone), Ficoll, Dextran Sulfate e.t.c. (Wahl et al, Proc. Natl. Acad. Sci. 76, 3683-3687, USA, 1979). The test strip assay system is deposited in a vessel containing the hybridization solution or assay development solution at room temperature and after 15 to 20 min the most a visible signal is generated and read out at the membrane area. The development of two red or purple-red lanes or dots [positions (8) and (9) on the strip] indicates a positive detection assay of the target-DNA in the sample and successful assay completion on the strip, while the development of only one lane or dot [position (9) on the strip] indicates a negative detection assay of the target-DNA and successful assay completion on the test strip.

The dipping test strip assay system of the present invention can be converted to a lateral flow test system with a slight modification of the shape and the absorbent material of area (2) of the strip, which comes into contact with the sample or with the assay-development solution on the strip.

With the dipping test strip assay system of the present invention and the hybridization assay protocols developed on the strip of the present invention, it is possible to detect a specific nucleic acid sequence of at least 50 base pairs.

The use of the strip test system of the present invention must take place with a volume of at least 5 µL PCR product and preferably between 5µL-15 µL.

The following Examples are provided by way of illustration only.

### EXAMPLE 1

Hybridization assay development on the dipping test strip according to the protocol I, for the detection of the specific products of the ARMS PCR reaction which is used for the genetic diagnosis of the IVS-n110 point mutation in the b-globin gene causing beta-Thallasaemia, said PCR is performed with the use of genomic DNA as a template. The ARMS method (Amplification Refractory Mutation System) is a variation of the PCR reaction permitting the specific amplification of a segment of an allele from an individual's genomic DNA.The ARMS method is based on the PCR primer's attribute according to which when the oligonucleotide primers have a mismatch at their 3' end, they cannot anneal to the template so that the selected DNA fragment can be amplified to yield a PCR product. Primers are designed to anneal to the normal and the mutated DNA sequence. Two amplification reactions are prepared for each sample. In each reaction a common primer is used labeled with Biotin at its 5' end, and another primer complementary to the region suspected to carry the mutation. The second primer differs as far as the 3' end is concerned, in each reaction regarding the same sample: at the first one it anneals to the normal sequence, while at the second to the mutated. In order for the hybridization of the last two primers to be more specific to their complementary sequence in the DNA template, both the normal and the mutated primer have one additional base-mismatch 3 or 4 nucleotides from their 3' end. When an individual is homozygous regarding the mutation, the PCR reaction with the specific primers for the amplification of the normal sequence will not yield a PCR product. On the contrary, the PCR reaction with the specific primers for the amplification of the mutated sequence will provide a PCR product. For the detection of the products of the two PCR reactions performed for each sample, two dipping test strips are required. When the result of the hybridization assay on both strips for the same sample is positive, then the individual is heterozygous for the mutation. When the result of the hybridization assay is positive on only one of the two strips for the same sample, and particularly when the detection is positive only for the product of the PCR reaction that amplifies the normal sequence, then the individual does not carry the mutation. Finally, when the detection assay is positive only on one test strip for the same sample, and specifically the strip used for the product of the PCR reaction that amplifies the mutated sequence, then the individual is homozygous for said mutation.

A 20mer oligonucleotide-probe (17) of complementary sequence to a region of the Biotinylated strand of the specific PCR product is used. Said probe carries additionally 80-100 Thymines (T) at it's 3' or 5' end.

For the construction of the dipping test strip assay system the following procedure is necessary:

0,5 µg of the protein Streptavidin (8) in a phosphate buffer 15 mM pH 7,2, is immobilized on a Predator Laminated membrane (Pall & Gelman, USA), as well as 500 fmol of an oligonucleotide (9) consisting of at least 80 Thymines (T), both having the shape of a dot, at a distance of 1cm and 1,5 cm from the lower edge of the membrane respectively. The immobilization takes place by application of each reagent on the selected region of the membrane, followed by baking at 70 °C for 30 min in an oven of stable temperature with limited humidity. After that, the system of the dipping strip is assembled on a flexible polystyrene film. An oligonucleotide consisting of 100 Adenines (A), modified with an SH- group at it's 5' end, is conjugated on the surface of colloidal gold particles of diameter 40 nm by adding it at a quantity of the particles in a ratio of: (moles of gold particles)/(moles of oligonucleotide)=1/500, followed by incubation at 4 °C for 56 hours. Consequently, the conjugated colloidal gold particles' solution is concentrated ten fold and the particles are brought to a solution containing Bovine Serum Albumin (BSA) 5% w/v, Sucrose 5% w/v, 0,05% v/v Tween 20 and they are allowed to air dry after being deposited on area (5) of the test strip system. Approximately 9x10⁹-1,1x10¹⁰ conjugated gold particles are dried on each dipping test strip system.

Heating at 95 °C for 5 min denatures the Biotinylated PCR product of the particular ARMS reaction (435 bp), which is then prehybridized with the of complementary sequence oligonucleotide-probe (17) by incubation for at least 15-20 min at a temperature depending on the melting point (Tm) of the probe which was 42 °C for this particular case and in a maleic acid buffer containing 100mM salt, pH 7,5. For the prehybridization of 5 µL PCR product (100-200 fmol/µL), 2000 fmol of the probe is used and the total volume of the prehybridization solution is 15 µL (preferably 15-20 µL). After that, the prehybridized PCR product is deposited at area (4) of the dipping test strip which is immersed in 300 µL of hybridization solution (or assay development solution) that contains PBS buffer pH 7,5-7,6, Bovine Serum Albumin (BSA) 5%, NaCl 150 mM, 20% glycerol and 0,5% Tween 20. The test strip assay system is deposited in a vessel containing the hybridization solution or assay development solution at room temperature and after 15 to 20 min the most a visible signal is generated and read out at the membrane area. The development of two red or purple-red lanes or dots [positions (8) and (9) on the strip] indicates a positive detection assay of the target-DNA in the sample and successful assay completion on the strip, while the development of only one lane or dot [position (9) on the strip] indicates a negative detection assay of the target-DNA and successful assay completion on the test strip.

### EXAMPLE 2

Hybridization assay development on the dipping test strip according to the protocol II, for the detection of the specific products of the nested PCR reaction, which is used for the amplification of the specific DNA sequence of the Cytomegalovirous (CMV) from an individual's genomic DNA. The nested PCR reaction is used in order to significantly increase the PCR's sensitivity and specificity, particularly when the amplification of a rare sequence is desired (low-copy-number-nucleic acid template) and if a large number of cycles is used there is an increased risk of amplification of non specific products. Two consecutive amplification reactions take place with a different primer pair used in each reaction. The first PCR contains an external pair of primers used to amplify an about 300 bp fragment. The second PCR contains an internal pair of primers, which hybridize to the 300 bp fragment amplified by the first set of primers (external primers), and is used to amplify a 163 bp fragment. Two oligonucleotides-probes consisting of 20-24 bases of complementary sequence to two discernible areas of the same strand of the 163 bp PCR product are selected. One of said oligo-probes is labeled with Biotin (20) at its 3' end with the use of the enzyme: terminal transferase, while the other oligo-probe (21) is tailed with additional 80-100 Thymines (T) at it's 3' end with the use of said enzyme. The tailing procedure is described below: a) Labeling with Biotin: In an eppendorf a mixture is prepared containing 4µL 5xTdT Bufffer (labeling buffer containing: 200 mmol/L potassium cacodylate, Tris-HCl 50 mmol/L, Bovine Serum Albumin 0,4 mg/mL, pH 7,2), 1µL CoCl₂ 25mmol/L, 1µL probe (19) (100pmol/µL), 1µL dNTPs mix (without dATP) 10mM, 2,5 µL Biotin-14-dATP 0,4 mM, 1,2µL terminal transferase (30 Units) and distilled water to final volume 20 µL. The mixture is incubated at 37°C for one hour. The reaction is terminated by the addition of 2,5µL EDTA 0,4 M, and the tailed oligo-probe is purified from the excess of the reagents used with the use of a Sephadex G-25 column (CPG, USA). b) Tailing with a number of Thymines (T): In an eppendorf a mixture is prepared containing 4µL 5xTdT Bufffer (labeling buffer containing: 200 mmol/L potassium cacodylate, Tris-HCl 50 mmol/L, Bovine Serum Albumin 0,4 mg/mL, pH 7,2), 1µL CoCl₂ 25mmol/L, 1µL probe (20) (100pmol/µL), 1µL dATP 10mM, 1,2µL terminal transferase (30 Units) and distilled water to final volume 20 µL. The mixture is incubated at 37°C for one hour. The reaction is terminated by the addition of 2,5µL EDTA 0,4 M.

The procedure for the assembly of the dipping test strip assay system is the same as the one described in the EXAMPLE 1.

Heating at 95°C for 5 min denatures the 163bp PCR product of the particular PCR reaction, which is then prehybridized with the two of complementary sequence oligonucleotides-probes (20,21) by incubation for at least 15-20 min at a temperature depending on the melting point (Tm) of the probes which was 37 °C for this particular case and in a PCR buffer containing 50mM salt, pH 7,5. For the prehybridization of 5 µL PCR product (100-200 fmol/µL), 2000 fmol of the probe is used and the total volume of the prehybridization solution is 15 µL (preferably 15-20 µL). After that, the prehybridized PCR product is deposited at area (4) of the dipping test strip which is immersed in 300 µL of hybridization solution (or assay development solution) that contains PBS buffer pH 7,5-7,6, Bovine Serum Albumin (BSA) 5%, NaCl 150 mM, 20% glycerol and 0,5% Tween 20. The test strip assay system is deposited in a vessel containing the hybridization solution or assay development solution at room temperature and after 15 to 20 min the most a visible signal is generated and read out at the membrane area. The development of two red or purple-red lanes or dots [positions (8) and (9) on the strip] indicates a positive detection assay of the target-DNA in the sample and successful assay completion on the strip, while the development of only one lane or dot [position (9) on the strip] indicates a negative detection assay of the target-DNA and successful assay completion on the test strip.

## Claims

1. A dipping test strip assay system for the qualitative and/or quantitative one step determination of a specific nucleic acid sequence (16, 19) in a liquid sample, comprising:
A) a test strip (1), said test strip (1) having four distinct areas:
a) a first area (2), which contacts said sample or assay-development solution;
b) a second area (3) onto which a visible signal producing substance is dried;
c) a third area (6) for signal development and assay completion control, comprising a first zone (8) containing immobilized streptavidin and a second zone (9) containing an immobilized oligonucleotide; and
d) a fourth area (7) to withhold the excess of said liquid sample;
**characterized in that**
said visible signal producing substance consists of colloidal gold particles (14) conjugated with an oligonucleotide (15) of a certain length and having a defined nucleotide sequence and can be solubilized by the liquid sample or assay-development solution;
said immobilized oligonucleotide (12) in the second zone (9) of said third area (6) of the test strip (1) has a nucleotide sequence complementary to the nucleotide sequence of said oligonucleotide (15) conjugated with said colloidal gold particles (14);
and **in that**
said dipping test strip assay system comprises further:
B) a distinct reagent (17; 20, 21) consisting of one or more oligonucleotides that hybridize specifically with said nucleic acid and that confer to said hybridized nucleic acid the ability to simultaneously hybridize to said oligonucleotide (15) that is conjugated with said colloidal gold particles (14) and to bind to said immobilized streptavidin of the first zone (8) of said third area (6).

2. A dipping test strip assay system according to claim 1, further **characterized in that** said visible signal producing substance is dried at the lower half (5) of said second area (3) of said test strip (1).

3. A dipping test strip assay system according to claim 1, **characterized in that** said nucleic acid to be assayed is biotinylated and said reagent (17; 20, 21) consists of one oligonucleotide (17) comprising a first part having a sequence complementary to a region of the nucleotide sequence of said biotinylated nucleic acid (16) and a second part having a nucleotide sequence complementary to the defined nucleotide sequence of said oligonucleotide (15) conjugated with said colloidal gold particles (14).

4. A dipping test strip assay system according to claim 1, **characterized in that** said nucleic acid to be assayed is not biotinylated and said reagent (17; 20, 21) comprises:
a first oligonucleotide (20) having a sequence complementary to a region of the nucleotide sequence of said nucleic acid (19) and is bound at one of its ends to a biotin molecule; and
a second oligonucleotide (21) comprising a first part having a sequence complementary to a region of the nucleotide sequence of said nucleic acid (19) and a second part having a nucleotide sequence complementary to the defined nucleotide sequence of said oligonucleotide (15) conjugated with said colloidal gold particles (14).

5. A dipping test strip assay system according to any one of the preceding claims, **characterized in that** said defined nucleotide sequence that is conjugated with said colloidal gold particles (14) has a length of between 60 and 100 bases.

6. A dipping test strip assay system according to any one of the preceding claims, **characterized in that** said defined nucleotide sequence that is conjugated with said colloidal gold particles (14) consists of 20 random nucleotides followed by 40-80 thymine bases.

7. A dipping test strip assay system according to any one of claims 1-6, **characterized in that** said defined nucleotide sequence that is conjugated with said colloidal gold particles (14) consists of 20 random nucleotides followed by 40-80 adenine bases.

8. A dipping test strip assay system according to any one of the preceding claims, **characterized in that** said visible signal producing substance (14, 15) is dried onto said second area (3) of said test strip (1) by (i) modifying the 5'-end of said oligonucleotide (15) with a defined nucleotide sequence with an SH group, (ii) adding said oligonucleotide in a conjugation solution containing said colloidal gold particles in a mole ratio (colloidal gold particles)/(oligonucleotide) of at least 1/500 and incubating for a sufficient time for the conjugation between said oligonucleotide and said gold particles to take place, (iii) dispersing said conjugated particles in a solution and applying said solution to said test strip at said second area (3), and (iv) drying said applied solution of conjugated particles onto said second area (3) of said test strip (1) by baking or by air-drying.

9. A dipping test strip assay system according to claim 8, further **characterized in that** said solution applied to said test strip at said second area (3) in step (iii) contains Bovine Serum Albumin (BSA) 5%w/v, Sucrose 5%, 0,001%-0,1% v/v of a non-ionic surfactant or detergent such as Tween 20.

10. Method for the qualitative and/or quantitative one step determination of a specific nucleic acid sequence (16, 19) in a liquid sample using a dipping test strip assay system according to any one of the preceding claims, wherein said method comprises:
diluting said liquid sample in a prehybridization solution having dissolved therein said distinct reagent (17; 20, 21);
heating said prehybridization solution and allowing hybridization to take place;
applying said prehybridization solution onto said test strip (1);
immersing said first area (2) of said test strip (1) in a hybridization solution or assay development solution, and
allowing sufficient time for the second zone (9) of said third area (6) of the test strip to become colored, at which time the presence and/or the intensity or the absence of color at said first zone (8) of said third area (6) of the test strip indicates the presence and/or the quantity or the absence of said nucleic acid sequence (16, 19) in the liquid sample.

11. Method according to claim 10 and using a dipping test strip assay system according to any one of the preceding claims, further **characterized in that** said pre-hybridization solution is applied onto the upper half (4) of said second area (3) of said test strip (1).

12. Method according to any of the claims 10, 11 and using a dipping test strip assay system according to claim 3, **characterized in that** said nucleic acid (16) to be assayed has been previously biotinylated.

13. Method according to any of the claims 10-12 further **characterized in that** said hybridization or assay development solution contains PBS buffer pH 7,5-7,6, Bovine Serum Albumin (BSA) 5%, NaCl at least 150mM, glycerol at least 15% and at least 0,1% of a non-ionic surfactant or detergent such as Tween 20.

14. Use of the dipping test strip assay system according to any one of claims 1-9 or of the method of any one of claims 10-13 for the detection and/or determination of specific nucleic acid sequences in a sample, which are indicative of a disease.

15. Use according to claim 14, **characterized in that** said specific nucleic acid sequences are viral or bacterial DNA and their detection is indicative of an infectious disease.

16. Use according to claim 14, **characterized in that** said specific nucleic acid sequences are mutated DNA sequences and their detection is indicative of a genetically transmitted disease.

## Patentansprüche

1. Test-Streife-Probesystem zur einstufigen qualitativen und/oder quantitativen Bestimmung einer spezifischen Nukleinsäuresequenz (16, 19) in einer flüssigen Probe, aufweisend:
A) eine Test-Streife (1), wobei die genannte Test-Streife (1) vier unterschiedliche Regionen hat:
a) eine erste Region (2), die im Kontakt mit der genannten Probe oder Bestimmungentwicklungslösung steht,
b) eine zweite Region (3), auf die eine visualle Signale produzierende Substanz getrocknet ist,
c) eine dritte Region (6) zur Signalentwicklung and Kontrollierung des Bestimmungsabschlusses, die eine erste Zone (8) enthaltend immobilisiertes Streptavidin und eine zweite Zone (9) enthaltend ein immobiliziertes Oligonukleotid aufweist, and
d) eine vierte Region (7) zum Zurückhalten der überschüssigen flüssigen Probe,
**dadurch gekennzeichnet, daß**
die visualle Signale produzierende Substanz aus kolloidalen Goldteilchen (14) besteht, die mit einem Oligonukleotid (15) von spezifischen Länge and enthaltend eine spezifische Nucleotidsequenz konjugiert sind and von der flüssigen Probe oder Bestimmungentwicklungslösung solubilisiert werden können,
das genannte immobilisierte Oligonukleotid (12) in der zweiten Zone (9) der genannten dritten Region (6) der Test-Streife (1) eine Nukleotidsequenz hat, die mit der Nukleotidsequenz des genannten, mit den kolloidalen Goldteilchen (14) konjugierten Oligonukleotids (15) komplementär ist,
und daß das Test-Streife-Probesystem im weiteren aufweist:
B) ein underschiedliches Reagenz (17, 20, 21), bestehend aus einem oder mehreren Oligonukleotiden, die zu der genannten Nukleinsäure selektiv hybridisiert werden and gleichzeitig die genannte hybridisierte Nukleinsäure zum genannten, mit den kolloidalen Goldteilchen (14) konjugierten Oligonukleotid (15) hybridisieren lassen und zu dem genannten immobilizierten Streptavidin der ersten Zone (8) der genannten dritten Region (6) binden lassen.

2. Test-Streife-Probesystem gemäß Anspruch 1, **dadurch gekennzeichnet, daß** die genannte visualle Signale produzierende Substanz an der unteren Hälfte (5) der genannten zweiten Region (3) der genannten Test-Streife (1) getrocknet ist.

3. Test-Streife-Probesystem gemäß Anspruch 1, **dadurch gekennzeichnet, daß** die genannte zu testende Nukleinsäure biotinyliert ist and das genannte Reagenz (17, 20, 21) aus einem Oligonukleotid (17) aufweisend einen ersten Teil mit einer Sequenz komplementär zu einer Region der Nukleotidsequenz der genannten biotinylierten Nukleinsäure (16) und einen zweiten Teil mit einer Nukleotidsequenz komplementär zu der definierten Nukleotidsequenz des genannten Oligonukleotids (15), das mit den genannten kolloidalen Goldteilchen (14) konjugiert ist, besteht.

4. Test-Streife-Probesystem gemäß Anspruch 1, **dadurch gekennzeichnet, daß** die genannte zu testende Nukleinsäure nicht bionylisiert ist and das genannte Reagenz (17, 20, 21) aufweist:
ein erstes Oligonukleotid (20), das eine Sequenz komplementär zu einer Region der Nukleotidsequenz der genannten Nukleinsäure (19) hat und an der einer von seinen Enden zu einem Biotinmolekül gebunden ist, und
ein zweites Oligonukleotid (21) aufweisend einen ersten Teil mit einer Sequenz komplementär zu einer Region der Nukleotidsequenz der genannten Nukleinsäure (19) and einen zweiten Teil mit einer Nukleotidsequenz komplementär zur der definierten Nukleotidsequenz des genannten Oligonukleotids (15), das mit den genannten kolloidalen Goldteilchen (14) konjugiert ist.

5. Test-Streife-Probesystem gemäß einem der vorhergehenden Ansprüche, **dadurch gekennzeichnet, daß** die genannte definierte Nukleotidsequenz, die mit den genannten kolloidalen Goldteilchen (14) konjugiert ist, eine Länge zwischen 60 and 100 Basen hat.

6. Test-Streife-Probesystem gemäß einem der vorhergehenden Ansprüche, **dadurch gekennzeichnet, daß** die genannte definierte Nukleotidsequenz, die mit den genannten kolloidalen Goldteilchen (14) konjugiert ist, aus 20 zufälligen Nukleotiden besteht, die von 40-80 Thyminbasen gefolgt sind.

7. Test-Streife-Probesystem gemäß einem der Ansprüche 1-6, **dadurch gekennzeichnet, daß** die genannte definierte Nukleotidsequenz, die mit den genannten kolloidalen Goldteilchen (14) konjugiert ist, aus 20 zufälligen Nukleotiden besteht, die von 40-80 Adeninbasen gefolgt sind.

8. Test-Streife-Probesystem gemäß einem der vorhergehenden Ansprüche, **dadurch gekennzeichnet, daß** die genannte, visualle Signale produzierende Substanz (14, 15) auf die genannte zweite Region (3) der genannten Test-Streife (1) getrocknet wird durch (a) Modifizierung der 5'-Ende des genannten Oligonukleotids (15) mit einer definierten Nukleotidsequenz durch einer SH-Gruppe, (b) Zusetzen des genannten Oligonukleotids in eine Konjugierungslösung enthaltend die genannten kolloidalen Goldteilchen bei einer molar Verhältnis von (kolloidalen Goldteilchen)/(Oligonukleotid) zumindest von 1/500 and Inkubierung für genügende Zeit zur Konjugierung zwischen dem genannten Oligonukleotid and den genannten Goldteilchen, (c) Dispensierung der genannten konjugierten Goldteilchen in eine Lösung and Auftragen der genannten Lösung auf die genannte Test-Streife an der genannten zweiten Region (3), und (d) Trocknen der genannten aufgetragten Lösung von konjugierten Goldteilchen auf der genannten zweiten Region (3) der genannten Test-Streife (1) durch Backen oder Lufttrocknen.

9. Test-Streife Probesystem gemäß Anspruch 8, **dadurch gekennzeichnet, daß** die genannte Lösung, die auf die genannte Test-Streife an der genannten zweiten Region (3) bei der Stufe (c) aufgetragen wird, Rinder Serum Albumin (RSA) 5% w/v, Rohrzucker 5%, 0.001%-0.1% v/v eines nicht-ionogenen oberflächenaktiven Mitels bzw. Tensids e.g. Tween 20, enthält.

10. Verfahren zur einstufigen qualitativen und/oder quantitativen Bestimmung einer spezifischen Nukleinsäuresequenz (16, 19) in einer flüssigen Probe mit Hilfe eines Test-Streife Probesystems gemäss einem der vorhergehenden Ansprüche, wobei das genannte Verfahren aufweist:
Verdünnen der genannten flüssigen Probe in einer Vorhybridisierungslösung, in der das genannte underschiedliche Reagenz (17, 20, 21) gelöst ist;
Wärmen der genannten Vorhybridisierungslösung und Entwicklung der Hybridisierung;
Auftragen der genannten Vorhybridisierungslösung auf die genannte Test-Streife (1);
Eintauchen der genannten ersten Region (2) der genannten Test-Streife (1) in eine Vorhybridisierungslösung oder Bestimmungentwicklungslösung,
die zweite Zone (9) der genannten dritten Region (6) der Test-Streife für genügende Zeit sich färben lassen, wobei die Anwesenheit und/oder die Intensität oder die Abwesenheit von Farbe an der genannten ersten Zone (8) der genannten dritten Region (6) der Test-Streife auf die Anwesenheit und/oder die Menge oder die Abwesenheit von der genannten Nukleinsäuresequenz (16, 19) in der Probe hinweist.

11. Verfahren gemäß Anspruch 10 mit Hilfe eines Test-Streife-Probesystems gemäß einem der vorhergehenden Ansprüche, **dadurch gekennzeichnet, daß** die genannte Vorhybridisierungslösung auf die obere Hälfte (4) der genannten zweiten Region (3) der genannten Test-Streife (1) auftragen wird.

12. Verfahren gemäß einem der Ansprüche 10, 11 mit Hilfe eines Test-Streife-Probesystems gemäß Anspruch 3, **dadurch gekennzeichnet, daß** die genannte zu testende Nukleinsäure (16) biotinyliert ist.

13. Verfahren gemäß einem der Ansprüche 10-12 **dadurch gekennzeichnet, daß** die genannte Hybridisierungslösung bzw. Bestimmungentwicklungslösung PBS-Puffer pH 7.5-7.6, Rinder Serum Albumin (RSA) 5%, NaCl zumindest 150 mM, Glycerin zumindest 15%, und zumindest 0.1% eines nicht-ionogenen oberflächenaktiven Mitels bzw. Tensids e.g. Tween 20, enthält.

14. Verwerdung des Test-Streife-Probesystems gemäß einem der Ansprüche 1-9, oder des Verfahrens gemäß einem der Ansprüche 10-13 zum Nachweis und/oder zur Bestimmung von spezifischen Nukleinsäuresequenzen in einer Probe, der bzw. die auf eine Erkrankung hinweist.

15. Verwendung gemäß Anspruch 14, **dadurch gekennzeichnet, daß** die genannten spezifischen Nukleinsäuresequenzen virale oder bakterielle DNA sind, und ihr Nachweis auf eine infektiöse Erkrankung hinweist.

16. Verwendung gemäß Anspruch 14, **dadurch gekennzeichnet, daß** die genannten spezifischen Nukleinsäuresequenzen mutierte DNA Sequenzen sind, und ihr Nachweis auf eine genetisch übertragbare Erkrankung hinweist.

## Revendications

1. Système d'essai à bandelette réactive trempant pour la détermination qualitative et/ou quantitative en une étape d'une séquence d'acide nucléique particulière (16, 19) dans un échantillon liquide, comprenant:
A) une bandelette réactive (1), laquelle bandelette réactive (1) a quatre aire distinctes:
a) une première aire (2), laquelle fait contact avec ledit échantillon ou solution de développement de l'essai;
b) une deuxième aire (3) sure laquelle est séchée une substance produisant un signal visible;
c) une troisième aire (6) pour le développement du signal et le contrôle d'achèvement de l'essai, comprenant une première zone (8) contenant de la streptavidine immobilisée et une deuxième zone (9) contenant un oligonucléotide immobilisé; et
d) une quatrième aire (7) pour retenir l'excès dudit échantillon liquide;
**caractérisé en ce que**
ladite substance produisant un signal visible est constituée de particules d'or colloïdal (14) conjuguées à un oligonucléotide (15) d'une longueur spécifique et ayant une séquence nucléotidique déterminée et peut être solubilisée par l'échantillon liquide ou ladite solution de développement de l'essai;
ledit oligonucléotide (12) immobilisé dans la deuxième zone (9) de ladite troisième aire (6) de la bandelette réactive (1) a une séquence nucléotidique complémentaire de la séquence nucléotidique dudit oligonucléotide (15) qui est conjugué audites particules d'or colloïdal (14);
et **en ce que**
ledit d'essai à bandelette réactive trempant comprend en plus:
B) un réactif distinct (17; 20, 21) consistant en un ou plusieurs oligonucléotides lesquelles s'hybrident spécifiquement avec ledit acide nucléique et confèrent audit acide nucléique hybridé l'aptitude de s'hybrider simultanément avec ledit oligonucléotide (15) qui est conjugué audites particules d'or colloïdal (14) et de s'attacher à ladite streptavidine immobilisée de la première zone (8) de ladite troisième aire (6).

2. Système d'essai à bandelette réactive trempant selon la revendication 1, **caractérisé en outre en ce que** ladite substance produisant un signal est séchée sur la moitié inférieure (5) de ladite deuxième aire (3) de ladite bandelette réactive (1).

3. Système d'essai à bandelette réactive trempant selon la revendication 1, **caractérisé en ce que** ledit acide nucléique analysé est biotinylé et ledit réactif (17; 20, 21) est constitué d' un oligonucléotide (17) comprenant une première partie ayant une séquence complémentaire d' une région de la séquence nucléotidique dudit acide nucléique biotinylé (16) et une deuxième partie ayant une séquence nucléotidique complémentaire d'une séquence nucléotidique définie dudit oligonucléotide (15) qui est conjugué audites particules d'or colloïdal (14).

4. Système d'essai à bandelette réactive trempant selon la revendication 1, **caractérisé en ce que** ledit acide nucléique analysé n'est pas biotinylé et ledit réactif (17; 20, 21) comprend:
un premier oligonucléotide (20) ayant une séquence complémentaire d'une région de la séquence nucléotidique dudit acide nucléique (19) et lié par une des ses extrémités à une molécule de biotine; et
un deuxième oligonucléotide (21) comprenant une première partie ayant une séquence complémentaire d'une région de la séquence nucléotidique dudit acide nucléique (19) et une deuxième partie ayant une séquence nucléotidique complémentaire de la séquence nucléotidique définie dudit oligonucléotide (15) qui est conjugué audites particules d'or colloïdal (14).

5. Système d'essai à bandelette réactive trempant selon une quelconque des revendications précédentes, **caractérisé en ce que** ladite séquence nucléotidique définie qui est conjuguée audites particules d'or colloïdal (14) a une longueur de 60 à 100 bases.

6. Système d'essai à bandelette réactive trempant selon une quelconque des revendications précédentes, **caractérisé en ce que** ladite séquence nucléotidique définie qui est conjuguée audites particules d'or colloïdal (14) est constituée de 20 nucléotides quelconques suivis de 40-80 base de thymine.

7. Système d'essai à bandelette réactive trempant selon une quelconque des revendications 1-6, **caractérisé en ce que** ladite séquence nucléotidique définie qui est conjuguée audites particules d'or colloïdal (14) est constituée de 20 nucléotides quelconques suivis de 40-80 bases d'adénine.

8. Système d'essai à bandelette réactive trempant selon une quelconque des revendications précédentes, **caractérisé en ce que** ladite substance produisant un signal visible (14, 15) est séchée sur ladite deuxième aire (3) de ladite bandelette réactive (1) par (i) modification de l'extrémité 5' dudit oligonucléotide (15) à une séquence nucléotidique définie avec un groupement SH, (ii) addition dudit oligonucléotide à une solution de conjugaison contenant lesdites particules d'or colloïdal avec un rapport molaire (particules d'or colloïdal)/(oligonucléotide) au moins 1/500 et incubation pendant un temps suffisant pour l'accomplissement de la conjugaison entre ledit oligonucléotide et lesdites particules d'or, (iii) dispersion des dites particules conjuguées dans une solution et application de ladite solution sur ladite bandelette réactive à ladite deuxième aire (3), et (iv) séchage de ladite solution de particules conjuguées appliquée sur ladite deuxième aire (3) de ladite bandelette réactive (1) par étuvage ou par séchage dans l'air.

9. Système d'essai à bandelette réactive trempant selon la revendication 8, **caractérisé en outre en ce** ladite solution appliquée sur ladite bandelette réactive à ladite deuxième aire (3) dans l'étape (iii) contient 5% p/v d'albumine de sérum de veau (BSA), 5% sucrose, 0,001%-0,1% v/v d'un surfactant non ionique ou d'un détergent comme Tween 20.

10. Méthode pour la détermination qualitative et/ou quantitative en une étape d'une séquence d'acide nucléique particulière (16, 19) dans un échantillon liquide an utilisant un système d'essai à bandelette réactive trempant selon une quelconque des revendications précédentes, cette méthode comprenant:
la dilution dudit échantillon liquide dans une solution de pré-hybridation dans laquelle est dissout ledit réactif distinct (17; 20, 21);
le chauffage de ladite solution de pré-hybridation permettant à l'hybridation d'avoir lieu;
l'application de ladite solution de pré-hybridation sur ladite bandelette réactive (1);
l'immersion de ladite première aire (2) de ladite bandelette réactive (1) dans une solution d'hybridation ou dans une solution de développement de l'essai, et
l'attente pendant un temps suffisant pour que la deuxième zone (9) de ladite troisième aire (6) de la bandelette réactive soit colorée, moment auquel la présence et/ou l'intensité ou l'absence de couleur à cette première zone (8) de ladite troisième aire (6) de la bandelette réactive indique la présence et/ou la quantité ou l'absence de ladite séquence d'acide nucléique (16, 19) dans un échantillon liquide.

11. Méthode selon la revendication 10 et utilisant le système d'essai à bandelette réactive trempant selon une quelconque des revendications précédentes, **caractérisée en outre en ce que** ladite solution de pré-hybridation est appliquée sur la moitié supérieure (4) de ladite deuxième aire (3) de ladite bandelette réactive (1).

12. Méthode selon l'une quelconque des revendications 10, 11 et utilisant le système d'essai à bandelette réactive trempant selon la revendication 3, **caractérisé en ce que** ledit acide nucléique (16) analysé a été préalablement biotinylé.

13. Méthode selon l'une quelconque des revendications 10-12 **caractérisé en outre en ce que** ladite solution d'hybridation ou de développement de l'essai contient du tampon PBS à pH 7,5-7,6, de l'albumine de sérum de veau (BSA) 5%, au moins 150mM de NaCl, au moins 15% de glycérol et au moins 0,1% d' un surfactant non ionique ou d'un détergent comme Tween 20.

14. Utilisation du système d' essai à bandelette réactive trempant selon l'une quelconque des revendications 1-9 ou de la méthode d'une quelconque des revendications 10-13 pour la détection et/ou la détermination de séquences d' acide nucléique spécifiques dans un échantillon, lesquelles sont indicatives d'une maladie.

15. Utilisation selon la revendication 14, **caractérisée en ce que** lesdites séquences d'acide nucléique particulières sont de l'ADN viral ou bactérien et leur détection est indicative d'une maladie infectieuse.

16. Utilisation selon la revendication 14, **caractérisée en ce que** lesdites séquences d'acide nucléique particulières sont des séquences d'ADN mutées et leur détection est indicative d'une maladie transmise génétiquement.
